# EUROPEAN PATENT APPLICATION

(11) **EP 1 816 125 A1**
(43) Date of publication of application: **08.08.2007**
(21) Application number: 07101591.1
(22) Date of filing: 01.02.2007
(51) Int. Cl.: C07D 231/14, A61K 31/415, A61P 35/00

(54) **Novel crystalline forms of an antagonist of CB1 cannabinoid receptor and preparation method thereof**

(30) Priority: 02.02.2006 IN DE02842006
(71) Applicant: RANBAXY LABORATORIES, LTD., Gurgaon 12001 HR (IN)
(72) Inventor: Babu, Jayachandra, Surech, 122001 Haryana (IN); Chavan, Gajanan Jijaba, Shirur Beed, 413249 Maharashtra (IN); Khanduri, Chandra Has, 122017 Haryana (IN)
(74) Representative: Cronin, Brian Harold John

(57) **Abstract**

The present invention relates to several crystalline forms and an amorphous form of an antagonist of CB₁ cannabinoid receptor and methods for their preparation. The present invention is also directed towards pharmaceutical compositions comprising these polymorphs ofrimonabant. The invention provides method of treating obesity, smoking cessation, overweight and related diseases by administering to a patient in need thereof a therapeutically effective amount of the different polymorphic forms and the amorphous form of the current invention.

## Description

### Field of the Invention

The present invention relates to several crystalline forms and an amorphous form of 5-(4-chlorophenyl)-1-(2,4-dichlorophenyl)-4-methyl-N-1-piperidinyl-1H-pyrazole-3-carboxamide - a compound known generically as rimonabant (hereinafter called rimonabant), an antagonist of CB₁ cannabinoid receptor, and methods for their preparation. The present invention is also directed towards pharmaceutical compositions comprising the novel polymorphs of rimonabant. The invention provides methods of treating obesity, smoking cessation, overweight and related diseases by administering to a patient in need thereof a therapeutically effective amount of the novel polymorphs or the amorphous form.

### Background of the Invention

Rimonabant has a good affinity for the cannabinoid receptor and is therefore particularly valuable in therapeutic areas in which cannabis is traditionally known to be medicinally useful. The effects of cannabinoids are due to an interaction with specific high-affinity receptors present in the central nervous system *(*Devane et al., Molecular Pharmacology, 1988, 34, 605-613*)* and peripheral nervous system *(*Munro et al., Nature, 1993, 365, 61-65*).* Rimonabant is reportedly one of the more potent newer compounds currently undergoing clinical trials for the treatment of obesity, smoking cessation, overweight and related diseases.

The compound 5-(4-chlorophenyl)-1-(2,4-dichlorophenyl)-4-methyl-N-1-piperidinyl-1H-pyrazole-3-carboxamide (rimonabant), was first disclosed in European patent EP 0 656 354 (hereinafter referred to as '354 patent'). In this patent, the compound is isolated according to conventional techniques; more precisely, according to the embodiments exemplified, the compound is obtained after crystallization from isopropyl ether or by cooling of a medium containing the compound in methyl cyclohexane. The '354 patent makes no reference to the existence of crystalline or amorphous forms of the compound.

In U.S. Patent publication number 2005/0043356 A1, the crystalline form of rimonabant is obtained by following the '354 patent is termed as form I. The application discloses the existence of another crystalline form II of the compound which is prepared by dissolving rimonabant in hot state in a solvent chosen from methylcyclohexane in the pure state or containing 1 to 10% of water by volume, acetonitrile, 4-methyl-2-pentanone, acetone or a mixture of these solvents. As per this application, the said crystalline form I can be obtained directly from 5-(4-chlorophenyl)-1-(2,4-dichlorophenyl)-4-methylpyrazole-3-carboxylic acid, according to the method described in '354 patent; the acid is converted to its acid chloride by the reaction with thionyl chloride, and then the acid chloride is reacted with 1-aminopiperidine in the presence of triethylamine.

In the pharmaceutical industry there is a constant need to identify the critical physicochemical parameters that affect the drugs performance, stability, etc which play a key role in determining a drug's market acceptance and success. Polymorphism is one such parameter. It is well known that pharmaceutical substances can exhibit polymorphism. From a regulatory point of view there is an increasing demand on establishing the polymorphic characteristics, integrity and stability of a drug. Polymorphism is commonly defined as the ability of any substance to have two or more different crystal structures. Drug substances may also encapsulate solvent molecules when crystallized. These solvates or hydrates are referred to as pseudo polymorphs. In some cases, it is also possible that the amorphous form is the most desirable form. Different polymorphs, pseudo polymorphs or the amorphous form differ in their physical properties such as melting point, solubility etc. These can appreciably influence pharmaceutical properties such as dissolution rate and bioavailability. It is also economically desirable that the product is stable for extended periods of time without the need for specialized storage conditions. It is therefore important to evaluate polymorphism of drug substances. In addition, the discovery of new crystalline polymorphic forms of a drug enlarges the repertoire of materials that a formulation scientist has with which to design a pharmaceutical dosage form of a drug with a targeted release profile or other desired characteristics. Therefore, clearly, there is a need for developing various polymorphic forms o f rimonabant.

### Summary of the Invention

The inventors of the present invention undertook extensive experiments to study polymorphism issues associated with rimonabant and they have, surprisingly, obtained various hitherto unknown polymorphic forms of rimonabant These forms can be obtained from certain solvents under certain conditions and are herein designated as Form III, IV, V and amorphous.

Accordingly, the present invention is directed towards the polymorphic Forms III, IV, V and amorphous form of rimonabant compound and processes for the preparation of these polymorphs and the amorphous form.

### Description of the Drawings

The X-ray powder diffractograms (XRPD) were recorded on a PANalytical X' pert Pro instrument. The measurements were done using CuK radiation at 45kV.

The Differential Scanning Calorimetric (DSC) thermograms were recorded on a Mettler Toledo or Perkin Elmer Diamond DSC instrument

The Infra-red (IR) spectra were recorded on a Perkin Elmer Spectrum One FT-IR instrument using KBr pellets.

**Figure 1** depicts the XRPD of Form III of rimonabant.

**Figure 2** depicts the DSC of Form III of rimonabant.

**Figure 3** depicts the Infra-red spectrum (IR) of Form III of rimonabant.

**Figure 4** depicts XRPD of Form IV of rimonabant.

**Figure 5** depicts DSC of Form IV of rimonabant.

**Figure 6** depicts IR of Form IV of rimonabant.

**Figure 7** depicts XRPD of Form V ofrimonabant.

**Figure 8** depicts DSC of Form V ofrimonabant.

**Figure 9** depicts IR of Form V of rimonabant.

**Figure 10** depicts XRPD of amorphous form of rimonabant.

**Figure 11** depicts DSC of amorphous form of rimonabant.

**Figure 12** depicts IR of amorphous form ofrimonabant.

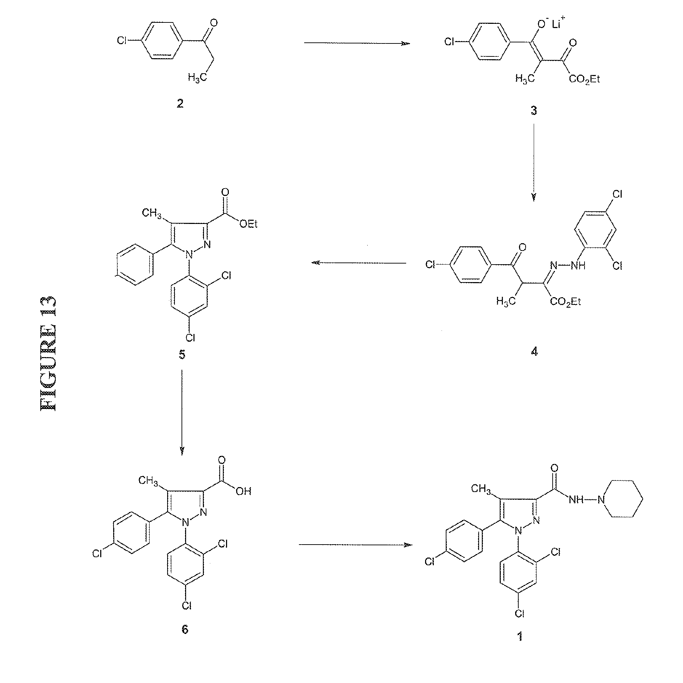

**Figure 13** depicts synthetic route for the preparation of rimonabant.

### Detailed Description of the Invention

The term "about" as used herein, when used along values assigned to certain measurements and parameters means a variation of 10% from such values, or in case of a range of values, means a 10% variation from both the lower and upper limits of such ranges. In case ofDSC and melting point data the term about means ±2°C.

A first aspect of the present invention provides a crystalline polymorph of rimonabant, herein designated as Form III, which exhibits an X-ray powder diffraction pattern with peaks expressed in d-values (Ǻ) and in 2θ angle as given in Table 1.

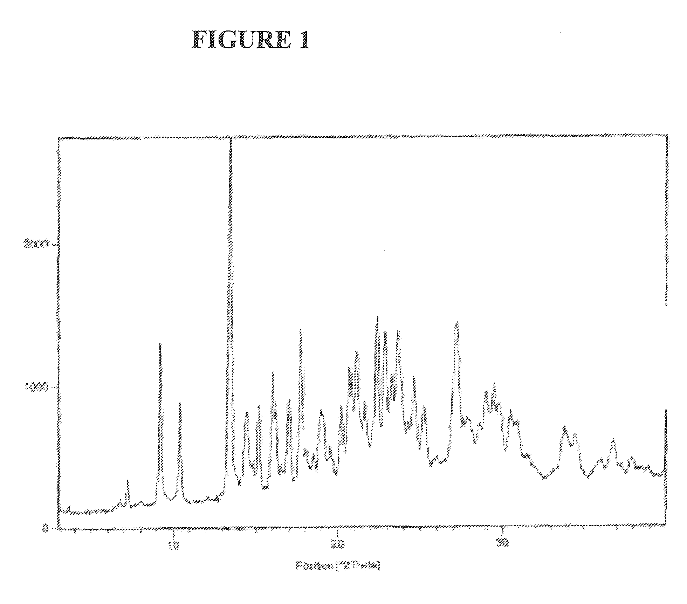

Crystalline Form III ofrimonabant is characterized by X-ray reflections at about 7.2218, 10.4594, 13.4718, 16.0555, 16.2819, 17.7165, 20.2555, 22.3736, 23.2326, 23.6070, 24.5675 and 29.4467 ± 0.2° 2θ, and further characterized by X-ray reflections at about 9.2488, 15.2155, 18.8909, 27.1696, 29.0166, and 29.8083 ± 0.2° 2θ. The representative XRPD pattern of Form III is given in Figure 1.

**Table 1: XRD peaks with d-spacings and 2θ angles for Form III ofrimonabant**

| **Angle (2θ)** | **d-values (Ǻ)** | **Angle (2θ)** | **d-values (Ǻ)** |
|---|---|---|---|
| 6.7069 | 13.17948 | 22.3736 | 3.97374 |
| 7.2218 | 12.2409 | 22.8223 | 3.8966 |
| 9.2488 | 9.56222 | 23.2326 | 3.82871 |
| 10.4594 | 8.45802 | 23.607 | 3.76883 |
| 13.4718 | 6.57273 | 24.5675 | 3.62363 |
| 14.4449 | 6.13209 | 25.2385 | 3.52879 |
| 15.2155 | 5.82321 | 27.1696 | 3.2822 |
| 16.0555 | 5.5204 | 27.9018 | 3.19771 |
| 16.2819 | 5.44414 | 29.0166 | 3.07734 |
| 16.9887 | 5.21919 | 29.4467 | 3.03337 |
| 17.7165 | 5.00639 | 29.8083 | 2.99739 |
| 18.5174 | 4.79163 | 30.4339 | 2.93718 |
| 18.8909 | 4.69772 | 30.9086 | 2.89315 |
| 19.1854 | 4.62628 | 33.831 | 2.64962 |
| 19.5406 | 4.54298 | 34.4258 | 2.6052 |
| 20.2555 | 4.38422 | 36.0155 | 2.49377 |
| 20.7276 | 4.28542 | 36.7693 | 2.44436 |
| 21.0503 | 4.22044 | 37.8839 | 2.37496 |
| 21.6062 | 4.1131 | | |

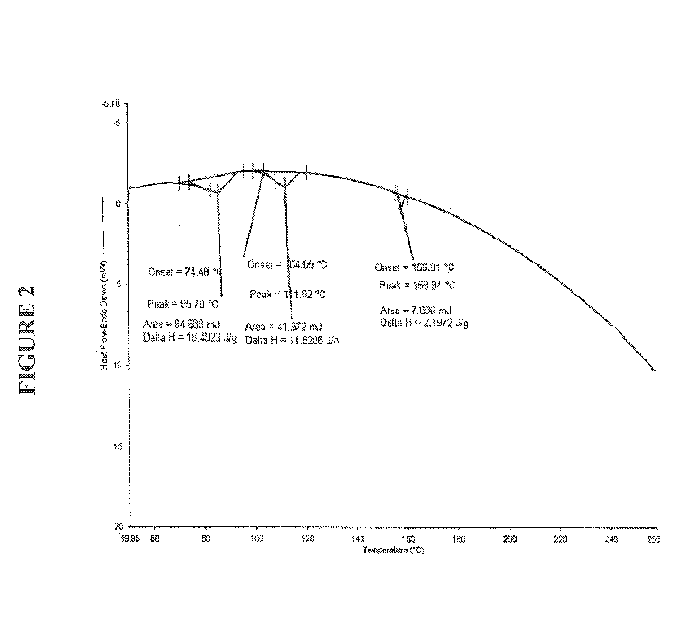

The polymorphic Form III has characteristic endotherms at about 85°C, about 112°C and about 158°C in the DSC thermogram. A representative DSC thermogram is given in Figure 2.

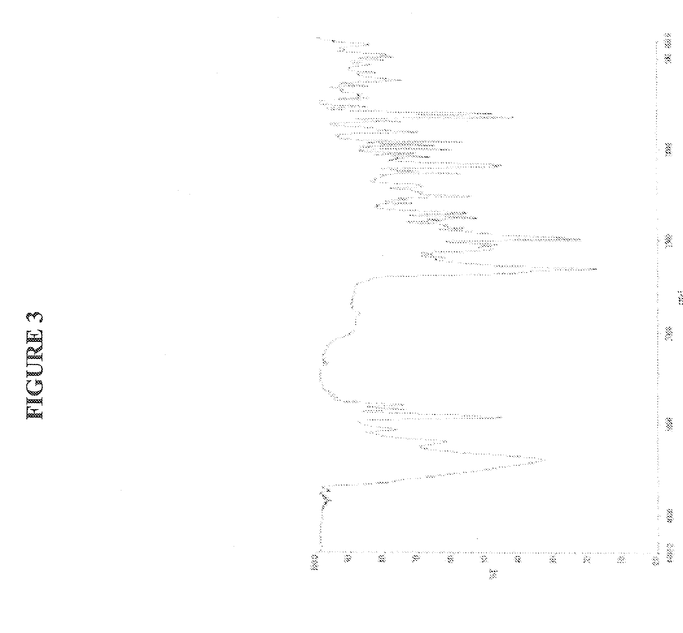

The IR spectrum of Form III is given in Figure 3.

A second aspect of the present invention provides a crystalline polymorph of rimonabant, herein designated as Form IV, which exhibits an X-ray powder diffraction pattern with peaks expressed in d-values (**Ǻ**) and in 2θ angle as given in Table 2.

Crystalline Form IV is the ethanol solvate of rimonabant.

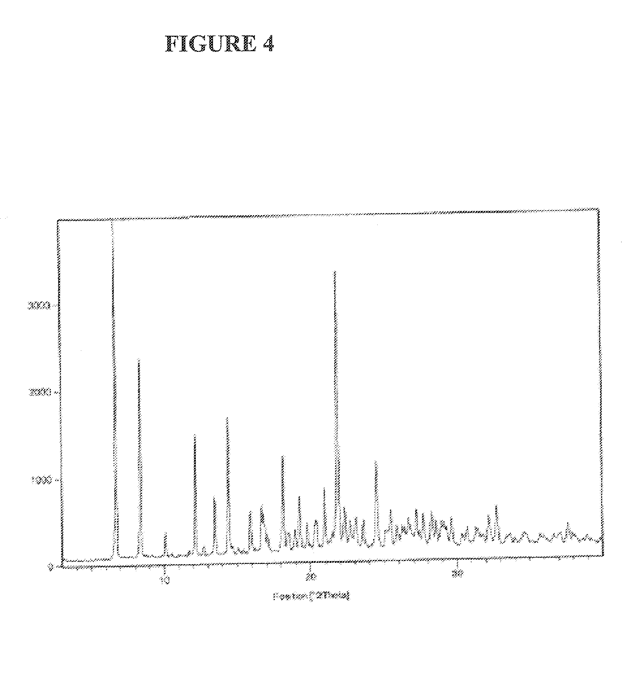

Form IV of rimonabant is characterized by X-ray reflections at 6.7539, 14.4343 and 21.8426 ± 0.2° 2θ and further characterized by X-ray reflections at 8.4449, 12.1877, 18.1892 and 24.4908 ± 0.2° 2θ. A representative XRPD pattern of Form IV of rimonabant is given in Figure 4.

**Table 2: XRD peaks with d-spacings and 2θ angles for Form IV of rimonabant**

| **Angle (2θ)** | **d-values (Ǻ)** | **Angle (2θ)** | **d-values (Ǻ)** |
|---|---|---|---|
| 6.7539 | 13.08788 | 23.5773 | 3.77352 |
| 8.4449 | 10.47055 | 24.4908 | 3.63479 |
| 10.1438 | 8.72042 | 25.4692 | 3.49735 |
| 12.1877 | 7.26219 | 26.6487 | 3.34516 |
| 13.5103 | 6.55411 | 27.1798 | 3.28099 |
| 14.4343 | 6.13657 | 27.6672 | 3.22429 |
| 15.9209 | 5.56675 | 28.2387 | 3.16032 |
| 16.6637 | 5.32024 | 28.5536 | 3.12618 |
| 18.1892 | 4.87735 | 28.9453 | 3.08476 |
| 18.535 | 4.78712 | 29.6107 | 3.01694 |
| 18.9822 | 4.67535 | 30.7079 | 2.9116 |
| 19.3371 | 4.59032 | 31.3675 | 2.85186 |
| 19.8275 | 4.47789 | 32.18 | 2.78169 |
| 20.4824 | 4.33617 | 32.7403 | 2.73536 |
| 20.9462 | 4.24119 | 33.6561 | 2.66299 |
| 21.8426 | 4.06912 | 34.6561 | 2.58841 |
| 22.3096 | 3.98499 | 35.8353 | 2.50589 |
| 22.6629 | 3.92366 | 37.6137 | 2.3914 |
| 23.0877 | 3.85242 | | |

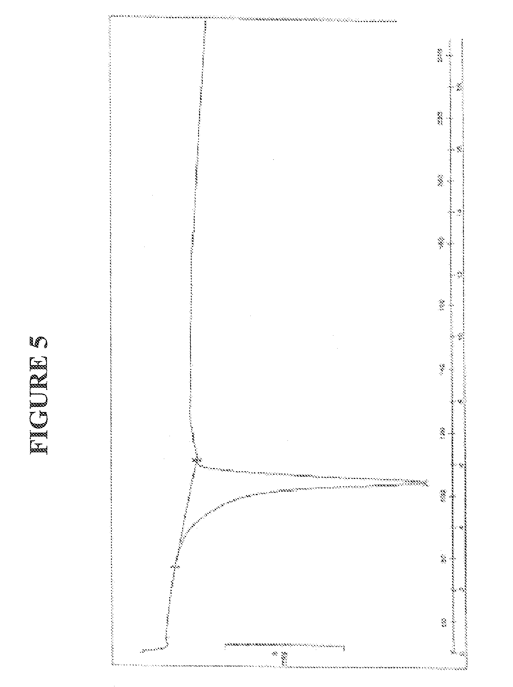

Form IV of rimonabant exhibits a characteristic endotherm at about 103°C in the DSC thermogram. A representative DSC thermogram is provided in Figure 5.

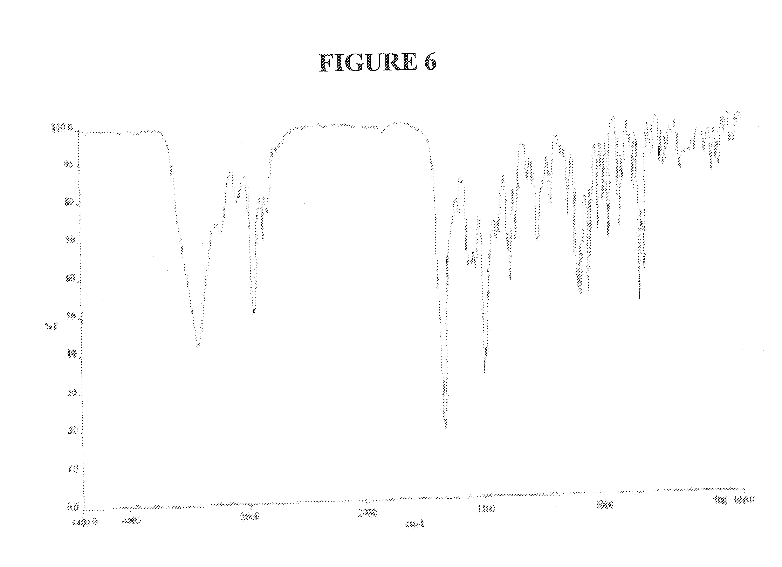

A representative IR spectrum of Form IV of rimonabant is provided in Figure 6.

A third aspect of the present invention provides crystalline polymorph of rimonabant, herein designated as Form V, which exhibits an X-ray powder diffraction pattern with peaks expressed in d-values (**Ǻ**) and in 2θ angle as given in Table 3.

Form V is the isopropyl alcohol solvate of rimonabant.

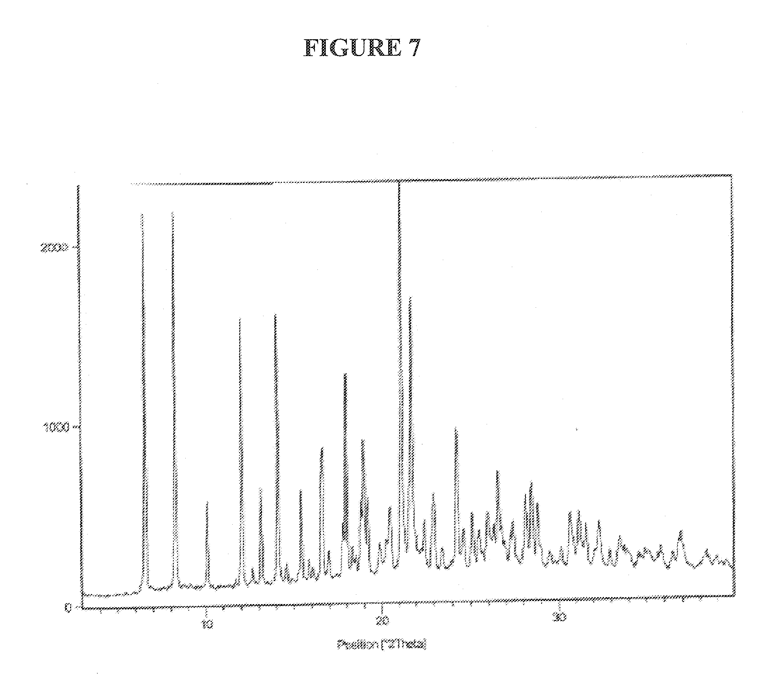

Form V of rimonabant is characterized by X-ray reflections at 8.3057, 12.0772, 13.1826, 14.1263, 17.9877, 19.0041, 21.1805, 24.1890, 26.5260 and 28.4417± 0.2° 2θ and further characterized by X-ray reflections at about 21.7665, 23.0255, and 28.1210 ± 0.2° 2θ. A representative XRPD pattern of Form V of rimonabant is provided in Figure 7.

**Table 3: XRD peaks with d-spacings and 2θ angles for Form V of rimonabant**

| **Angle (2θ)** | **d-values (Ǻ)** | **Angle (2θ)** | **d-values (Ǻ)** |
|---|---|---|---|
| 6.5892 | 13.41466 | 24.189 | 3.67946 |
| 8.3057 | 10.6457 | 24.5907 | 3.62026 |
| 10.1152 | 8.74506 | 25.0635 | 3.55302 |
| 12.0772 | 7.3284 | 25.962 | 3.43206 |
| 13.1826 | 6.71628 | 26.526 | 3.36036 |
| 14.1263 | 6.26964 | 27.3524 | 3.26067 |
| 15.4551 | 5.73347 | 28.121 | 3.17328 |
| 16.6544 | 5.32319 | 28.4417 | 3.13823 |
| 17.0057 | 5.21401 | 28.8148 | 3.09843 |
| 17.9877 | 4.93153 | 30.6537 | 2.91663 |
| 19.0041 | 4.67 | 31.1734 | 2.86918 |
| 19.2363 | 4.61415 | 31.5497 | 2.83581 |
| 19.9063 | 4.46033 | 32.3073 | 2.77102 |
| 20.5052 | 4.33139 | 33.4351 | 2.68009 |
| 21.1805 | 4.19479 | 35.8167 | 2.50715 |
| 21.7665 | 4.08318 | 36.8983 | 2.4361 |
| 22.4884 | 3.95371 | 38.3106 | 2.34949 |
| 23.0255 | 3.86269 | | |
| 23.4916 | 3.78708 | | |

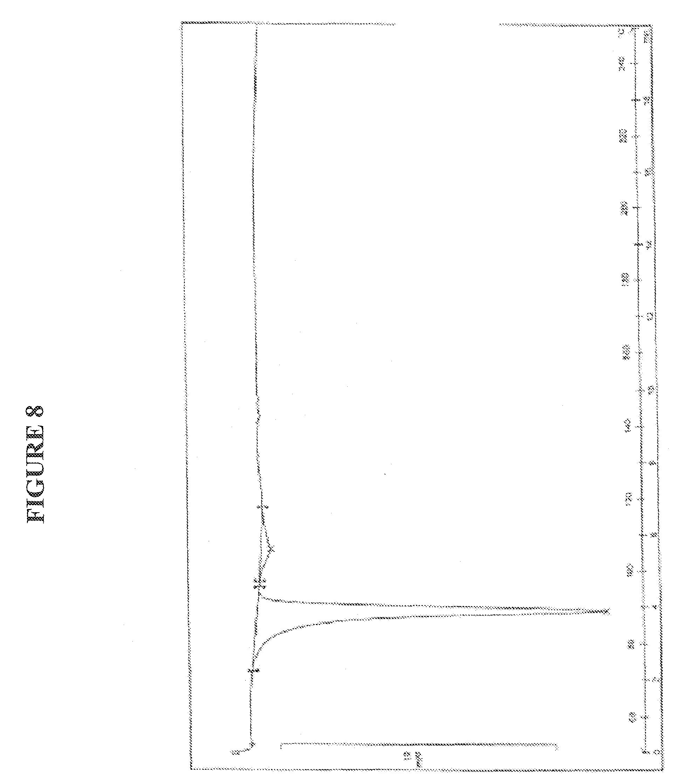

Form V ofrimonabant shows a characteristic endotherm at about 88°C and about 106°C in the DSC thermogram. A representative thermogram is provided in Figure 8.

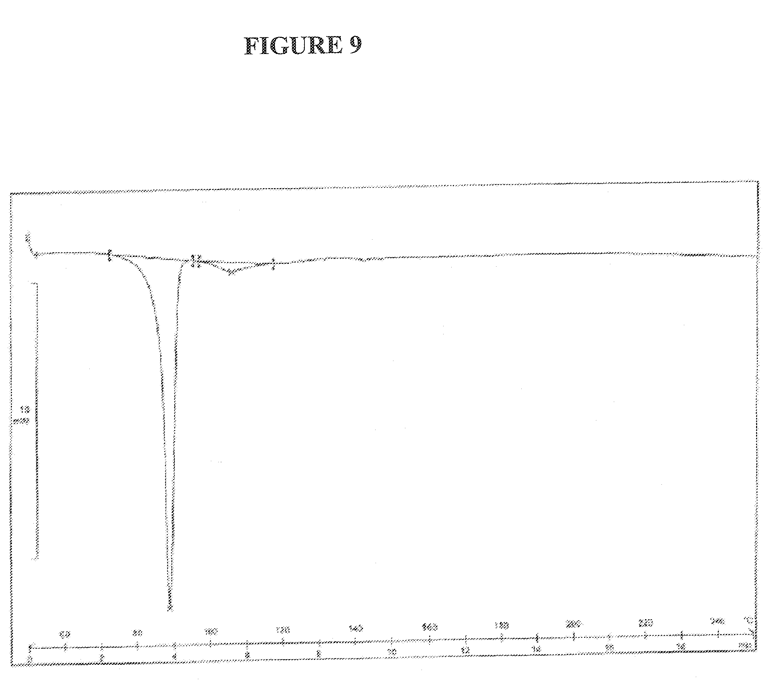

A representative IR spectrum of Form V ofrimonabant is provided in Figure 9.

A fourth aspect of the present invention provides the amorphous form ofrimonabant.

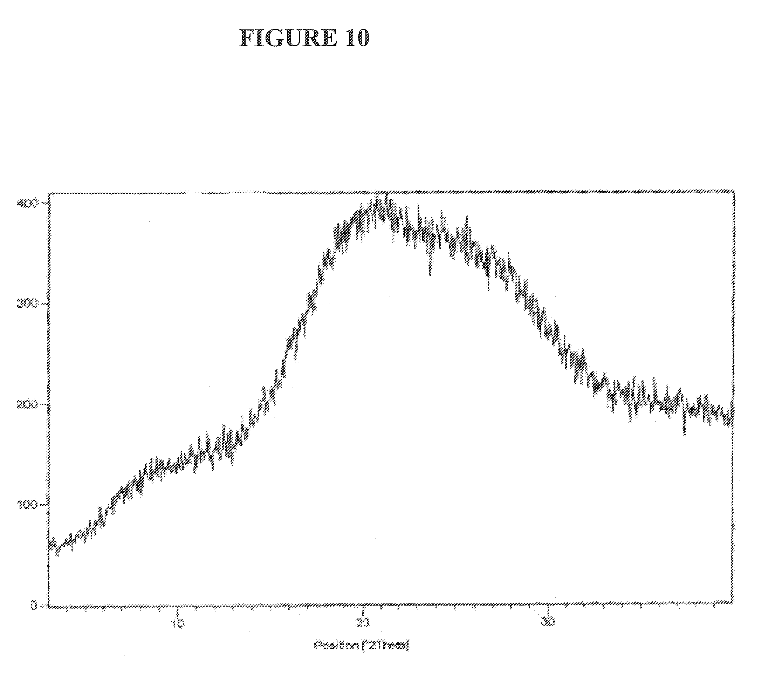

A representative XRPD pattern of amorphous rimonabant is provided in Figure 10.

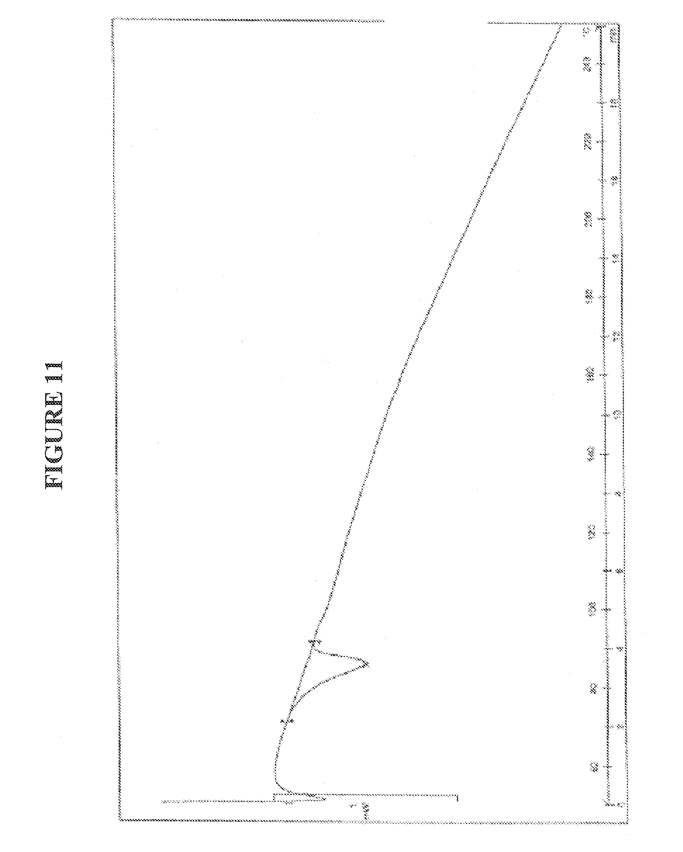

Amorphous rimonabant shows a characteristic endotherm at about 85°C in the DSC thermogram. A representative thermogram is provided in Figure 11.

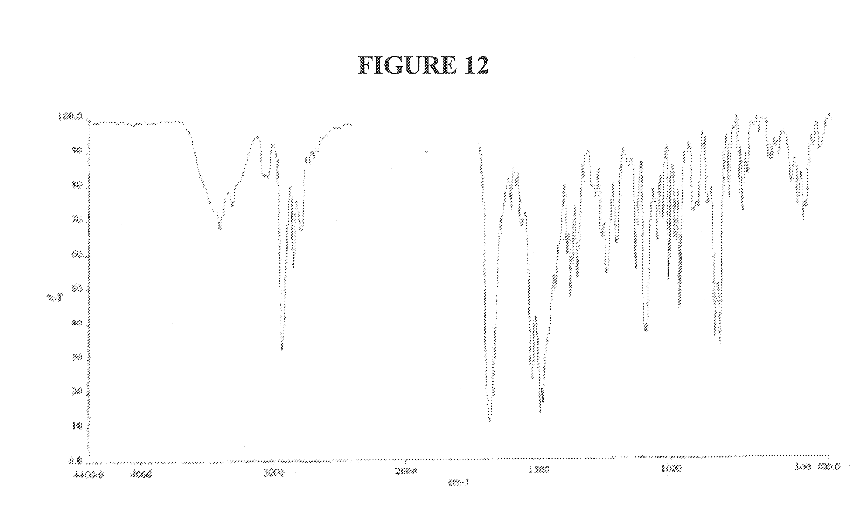

A representative IR spectrum of amorphous rimonabant is provided in Figure 12.

A fifth aspect of the present invention provides a process for the preparation of Form III of rimonabant which comprises crystallization of rimonabant from methanol, aqueous acetone or a mixture thereof.

Rimonabant, as used hereinabove in the fifth aspect as starting material, is prepared by any method known in the prior art and is present in polymorphic Form I, polymorphic Form II or a mixture thereof. Form IV, Form V, the amorphous form of rimonabant or a mixture thereof can also be used to prepare the said polymorphic Form III.

In an embodiment ofthis aspect, polymorphic Form I or Form II ofrimonabant or a mixture thereof is dissolved in methanol optionally through heating. The solution so obtained is treated with charcoal followed by cooling. The solution is then stirred for an hour at 15-25°C. The product so formed is isolated, washed with chilled methanol and dried under vacuum to get Form III of rimonabant.

In an another embodiment of this aspect, water is added to a solution of polymorphic Form I, Form II or a mixture thereof ofrimonabant in acetone to obtain polymorphic Form III. The solvent is evaporated from the solution and the residue is dried under vacuum at 60-75°C.

In an another embodiment of this aspect of the invention, the said polymorphic Form III can be obtained from polymorphic Form IV, V , the amorphous form of rimonabant or a mixture thereof by crystallizing them in methanol or aqueous acetone or a mixture thereof as the solvent. Form III of rimonabant can be obtained by evaporating the solvent from a solution of rimonabant in methanol or aqueous acetone or a mixture thereof

A sixth aspect of the present invention provides a process for the preparation of polymorphic Form I ofrimonabant which comprises crystallization ofrimonabant from isopropyl ether.

The said Form I can be prepared by crystallizing Form II, III, IV, V, the amorphous form of rimonabant or a mixture thereof from isopropyl ether.

Form II, III, IV, V, the amorphous form or a mixture thereofis dissolved in isopropyl ether at reflux temperature. The solution formed is treated with activated charcoal, filtered and then cooled to ambient temperature. The solution is then stirred for 2-3 hours at ambient temperature. The product so obtained is isolated and washed with isopropyl ether. It is then dried under vacuum to obtain said Form I.

Form I ofrimonabant can also be obtained by evaporating the solvent from a solution ofrimonabant in isopropyl ether.

A seventh aspect of the present invention provides a process for the preparation of Form IV ofrimonabant which comprises crystallization ofrimonabant from ethanol.

The said Form IV can be prepared by crystallizing the above mentioned Forms I, II, III, V, the amorphous form or a mixture thereof from ethanol.

Form I, II, III, V, the amorphous form or a mixture thereof is dissolved in ethanol optionally through heating. If required, the resulting solution is cooled to ambient temperature. The solution is then stirred for 2-3 hours. The product so obtained is isolated and washed with ethanol. It is then dried under vacuum to obtain said Form IV.

Form IV of rimonabant can also be obtained by evaporating the solvent from a solution of rimonabant in ethanol.

An eighth aspect of the present invention provides a process for the preparation of Form V ofrimonabant which comprises crystallization of rimonabant from isopropyl alcohol.

The said form V can be prepared by crystallizing the above mentioned Forms I, II, III, IV, amorphous form or mixture thereof from isopropyl alcohol.

Form I, II, III, IV, the amorphous form or a mixture thereof is dissolved in isopropyl alcohol optionally through heating. If required, the resulting solution is cooled to ambient temperature. The solution is then stirred for 1-2 hours. The product so obtained is isolated and washed with isopropyl alcohol. It is then dried under vacuum to obtain said polymorphic form V.

Form V of rimonabant can also be obtained by evaporating the solvent from a solution ofrimonabant in isopropyl alcohol.

A ninth aspect of the present invention provides a process for the preparation of rimonabant in an amorphous form which comprises treating rimonabant with a chlorohydrocarbon, aromatic hydrocarbon solvent or a mixture thereof and isolating said amorphous form from the reaction mixture thereof.

The said amorphous form can also be prepared by other techniques such as spray drying, roller drying, freeze drying, solvent precipitation etc.

Form I, II, III, IV or V as mentioned above or mixtures thereof is dissolved in a chlorohydrocarbon or aromatic hydrocarbon or a mixture thereof, optionally through heating. The solvent is recovered under vacuum. The material left is dried under vacuum to get amorphous form ofrimonabant.

The amorphous form of rimonabant can also be obtained by spray drying or roller drying or freeze drying or solvent precipitation of a solution of rimonabant in chlorohydrocarbon, aromatic hydrocarbon solvents or a mixture thereof.

The term chlorohydrocarbon used herein above in ninth aspect of this invention is referred to any chlorinated hydrocarbon solvent commonly known to a person who is skilled in the art. Preferably, the chlorinated solvent used herein is a chlorinated aliphatic hydrocarbon such as dichloromethane, 1,2-dichloroethane, etc.

The aromatic hydrocarbon used hereinabove in ninth aspect ofthis invention is referred to any aromatic solvent commonly known to a person who is skilled in the art such as toluene, xylenes, etc.

A tenth aspect of the present invention provides a pharmaceutical composition comprising Form III, IV, V, the amorphous form or a mixture thereof of rimonabant.

An eleventh aspect of the present invention provides a method of treating obesity, smoking cessation, overweight and related diseases which comprises of administering to a patient in need thereof a therapeutically effective amount of Form III, IV, V, the amorphous form, or a mixture thereof of rimonabant.

5-(4-Chlorophenyl)-1-(2,4-dichlorophenyl)-4-methyl-pyrazole-3-carboxylic acid and rimonabant were prepared by using the process described in US Patent 5,624,941 (example 154) which is incorporated by reference herein.

While the present invention has been described in terms of its specific aspects, certain modifications and equivalents will be apparent to those skilled in the art and are intended to be included within the scope of the present invention.

In the following section aspects are described by way of examples to illustrate the processes of the invention. However, these are not intended in any way to limit the scope of the present invention. Several variants of these examples would be evident to persons ordinarily skilled in the art.

### EXAMPLE 1

### Preparation of Form III of rimonabant

Thionyl chloride (2.84ml, 0.039mole) was added slowly to a suspension of 5-(4-chlorophenyl)-1-(2,4-dichlorophenyl)-4-methyl-pyrazole-3-carboxylic acid **(6)** (5g, 0.0139mole) in toluene (50ml) and the mixture was refluxed for 3h and evaporated under vacuum. The resultant acid chloride was dissolved in dichloromethane (45ml) and the solution was slowly added to a solution of 1-aminopiperidine (1.6ml, 0.0 1 6mole) and triethylamine (2ml) in dichloromethane (60ml) at 0-5°C. The reaction mixture was stirred at ambient temperature and the completion of the reaction was monitored by TLC. When the reaction was complete, as indicated by the near absence of the starting material, ice-cold water was added, the reaction mixture was stirred at ambient temperature for 15 min and the layers were separated. The organic layer was washed with water and brine solution. The solvent was evaporated under vacuum, methanol (25ml) was added and then the solvent (~10ml) was removed under vacuum. The residue was stirred for 1 hr at 15-20°C. The product was filtered, washed with chilled methanol (5ml) and dried at 40-45°C to get title product as crystalline Form-III (4g, 62%).

### EXAMPLE 2

### Preparation of Form III of rimonabant

### a. Preparation of rimonabant (1) (Form-I)

Thionyl chloride (3.72g, 0.031mole) was added slowly to a suspension of 5-(4-chlorophenyl)-1-(2,4-dichlorophenyl)-4-methyl-pyrazole-3-carboxylic acid **(6)** (4g, 0.01 1mole) in toluene (40ml) and the mixture was refluxed for 3 h. The solvent was evaporated off under vacuum. The resultant acid chloride was dissolved in dichloromethane (35ml) and the solution was slowly added to a solution of 1-aminopiperidine (1.22ml, 0.012mole) and triethylamine (1.55ml) in dichloromethane (44ml) at 0-5°C. The reaction mixture was stirred at ambient temperature and the completion of the reaction was monitored by TLC. When the reaction was complete, as indicated by the near absence of the starting material, ice-cold water was added, the reaction mixture was stirred at ambient temperature for 15 min and the layers were separated. Organic layer was washed with water and brine solution. The solvent was evaporated under vacuum, added hexanes (20ml) and stirred for 1 hr at ambient temperature. The product was filtered off, washed with hexanes (10ml) and dried at 40-45°C to get title product as crystalline Form-I (3g, 58%).

### b. Preparation of rimonabant (1) (Form-III)

Form I ofrimonabant (3g) was dissolved in hot methanol (30ml) . The resulting solution was treated with charcoal and it was then cooled to about 15°C. This solution was stirred for an hour at 15-20°C. The product was then filtered, washed with chilled methanol and dried under vacuum to get the title product (1.96g).

The title compound (Form III) can also be prepared by using Form II, IV, V, the amorphous form or mixtures thereof of rimonabant in place of Form I in the process described above.

### EXAMPLE 3

### Preparation of Form III of rimonabant

Form I of rimonabant (0.5g) was dissolved in acetone (5ml). Water (1ml) was added to the solution. The solvent was then evaporated and the residue was dried under vacuum at 65-70°C to obtain the title product (0.42 g).

The title compound (Form III) can also be prepared by using Form II, IV, V, the amorphous form or mixtures thereof of rimonabant in place of Form I in the process described above.

### EXAMPLE 4

### Preparation of Form I of rimonabant

Form III ofrimonabant (1g) was dissolved in isopropyl ether (35ml) and the solution was heated to reflux. This solution was treated with charcoal and then cooled to ambient temperature. The solution was then stirred for 2 hours at ambient temperature.

The product so obtained was filtered, washed with isopropyl ether and then dried under vacuum to obtain Form I ofrimonabant (0.48g).

The title compound (Form I) can also be prepared by using Form II, IV, V, the amorphous form or mixtures thereof of rimonabant in place of Form III in the process described above.

### EXAMPLE 5

### Preparation of Form IV of rimonabant

Form I ofrimonabant (10g) was dissolved in hot ethanol (100ml). The solution was then cooled to ambient temperature and stirred for 2 hours. The product so obtained was filtered, washed with ethanol and dried under vacuum to found the title product (8.75g).

The title compound (Form IV) can also be prepared by using Form II, III, V, the amorphous form or mixtures thereof of rimonabant in place of Form I in the process described above.

### EXAMPLE 6

### Preparation of Form V of rimonabant

Form I ofrimonabant (5g) was dissolved in hot isopropyl alcohol (25ml). The solution was then cooled to ambient temperature and stirred for about 1 hour. The product so obtained was filtered, washed with isopropyl alcohol and dried under vacuum to obtain the title product (4.4g).

The title compound (Form V) can also be prepared by using Form II, III, IV, the amorphous form or mixtures thereof of rimonabant in place of Form I in the process described above.

### EXAMPLE 7

### Preparation of amorphous form of rimonabant

Form I ofrimonabant (5g) was dissolved in hot dichloromethane (10ml). The solvent was then completely recovered under vacuum. The product so obtained was dried under vacuum to get amorphous form of rimonabant.
The title compound (amorphous form) can also be prepared by using Form II, III, IV, V or mixtures thereof of rimonabant in place of Form I in the process described above.

### EXAMPLE 8

### Preparation of amorphous form of rimonabant

Form I ofrimonabant (5g) was dissolved in hot toluene. The solvent was then completely recovered under vacuum. The product so obtained was dried under vacuum to get amorphous form ofrimonabant.

The title compound (amorphous form) can also be prepared by using Form II, III, IV, V or mixtures thereof of rimonabant in place of Form I in the process described above.

## Claims

1. A crystalline polymorph of rimonabant, denominated Form III, which is **characterized by** X-ray reflections at 7.2218, 10.4594, 13.4718, 16.0555, 16.2819, 17.7165, 20.2555, 22.3736, 23.2326, 23.6070, 24.5675 and 29.4467 ± 0.2° 2θ.

2. A crystalline polymorph of rimonabant, denominated Form IV, **characterized by** X-ray reflections at 6.7539, 14.4343 and 21.8426 ± 0.2° 2θ.

3. A crystalline polymorph of rimonabant, denominated Form V, **characterized by** X-ray reflections at 8.3057, 12.0772, 13.1826, 14.1263, 17.9877, 19.0041, 21.1805, 24.1890, 26.5260 and 28.4417 ± 0.2° 2θ.

4. A crystalline polymorph of claim 1 further **characterized by** X-ray reflections at about 9.2488, 15.2155, 18.8909, 27.1696, 29.0166, and 29.8083 ± 0.2° 2θ.

5. The crystalline polymorph of claim 1 or 4 having a powder X-ray diffraction pattern substantially as shown in Figure 1.

6. The crystalline polymorph of claim 1 or 4 having a DSC thermogram substantially as shown in Figure 2.

7. The crystalline polymorph of claim 1 or 4 having IR spectrum substantially as shown in Figure 3.

8. A crystalline polymorph of claim 2 further **characterized by** X-ray reflections at about 8.4449, 12.1877, 18.1892 and 24.4908 ± 0.2° 2θ.

9. The crystalline polymorph of claim 2 or 8 having a powder X-ray diffraction pattern substantially as shown in Figure 4.

10. The crystalline polymorph of claim 2 or 8 having a DSC thermogram substantially as shown in Figure 5.

11. The crystalline polymorph of claim 2 or 8 having IR spectrum substantially as shown in Figure 6.

12. The crystalline polymorph of claim 2 or 8 wherein the crystalline Form IV is an ethanol solvate.

13. A crystalline polymorph of claim 3 further **characterized by** X-ray reflections at about 21.7665, 23.0255, and 28.1210 ± 0.2° 2θ.

14. The crystalline polymorph of claim 3 or 13 having a powder X-ray diffraction pattern substantially as shown in Figure 7.

15. The crystalline polymorph of claim 3 or 13 having a DSC thermogram substantially as shown in Figure 8.

16. The crystalline polymorph of claim 3 or 13 having IR spectrum substantially as shown in Figure 9.

17. The crystalline polymorph of claim 3 or 13 wherein the crystalline Form V is an isopropyl alcohol solvate.

18. Rimonabant in amorphous form.

19. The amorphous form of claim 18 having a powder X-ray diffraction pattern substantially as shown in Figure 10.

20. The amorphous form of claim 18 having a DSC thermogram substantially as shown in Figure 11.

21. The amorphous form of claim 18 having IR spectrum substantially as shown in Figure 12.

22. A process for the preparation of crystalline polymorph Form III of rimonabant which comprises crystallization of rimonabant from methanol, aqueous acetone or a mixture thereof.

23. A process for the preparation of crystalline polymorph Form III of rimonabant which comprises
a) dissolving rimonabant in methanol, aqueous acetone or a mixture thereof.
b) optionally subjecting the solution of step a) to charcoal treatment
c) isolating crystalline rimonabant from the solution obtained in step a) or b)
d) optionally washing the crystalline compound of step c) with methanol, aqueous acetone or a mixture thereof.
e) drying crystalline rimonabant of step c) or d).

24. A process for the preparation of crystalline polymorph Form III of rimonabant which comprises evaporation of the solvent from a solution of rimonabant in methanol, aqueous acetone or a mixture thereof.

25. A process for the preparation of crystalline polymorph Form III of rimonabant which comprises
a) dissolving rimonabant in methanol, aqueous acetone or a mixture thereof.
b) optionally subjecting the solution of step a) to charcoal treatment
c) evaporating the solvent from the solution of step a) or b) to obtain crystalline compound
d) optionally washing the crystalline compound of step c) with methanol, aqueous acetone or a mixture thereof.
e) drying crystalline rimonabant of step c) or d).

26. Crystalline polymorph Form III of rimonabant obtained by the process of claims 22 to 25.

27. A process for the preparation of crystalline polymorph Form I of rimonabant which comprises crystallization or evaporation of the solvent from a solution of rimonabant in isopropyl ether.

28. A process for the preparation of crystalline polymorph Form I of rimonabant which comprises
a) dissolving rimonabant in isopropyl ether
b) optionally subjecting the solution of step a) to charcoal treatment
c) isolating the compound through crystallization or evaporation of the solvent from a solution of step a) or b) to obtain crystalline compound
d) optionally washing the crystalline compound of step c) with isopropyl ether
e) drying crystalline rimonabant of step c) or d).

29. Crystalline polymorph Form I of rimonabant obtained by the process of claim 27 or 28.

30. A process for the preparation of crystalline polymorph Form IV of rimonabant which comprises crystallization or evaporation of the solvent from a solution of rimonabant in ethanol.

31. A process for the preparation of crystalline polymorph Form IV of rimonabant which comprises
a) dissolving rimonabant in ethanol
b) optionally subjecting the solution of step a) to charcoal treatment
c) isolating the compound through crystallization or evaporation of the solvent from a solution of step a) or b) to obtain crystalline compound
d) optionally washing the crystalline compound of step c) with ethanol
e) drying crystalline rimonabant of step c) or d).

32. Crystalline polymorph Form IV of rimonabant obtained by the process of claim 30 or 31.

33. A process for the preparation of crystalline polymorph Form V of rimonabant which comprises crystallization or evaporation of the solvent from a solution of rimonabant in isopropyl alcohol.

34. A process for the preparation of crystalline polymorph Form V of rimonabant which comprises
a) dissolving rimonabant in isopropyl alcohol
b) optionally subjecting the solution of step a) to charcoal treatment
c) isolating the compound through crystallization or evaporation of the solvent from a solution of step a) or b) to obtain crystalline compound
d) optionally washing the crystalline compound of step c) with isopropyl alcohol
e) drying crystalline rimonabant of step c) or d).

35. Crystalline polymorph Form V of rimonabant obtained by the process of claim 33 or 34.

36. A process for preparing amorphous rimonabant comprising the step of spray drying or roller drying or freeze drying or solvent precipitation of a solution of rimonabant in one or more solvents selected from the group consisting of chlorohydrocarbons, aromatic hydrocarbons or a mixture thereof

37. A process for preparing amorphous rimonabant which comprises
a) treating rimonabant with one or more solvents selected from the group consisting of chlorohydrocarbons, aromatic hydrocarbons or a mixture thereof
b) isolating amorphous compound from the solution of step a).

38. The process of claim 36 or 37 wherein the chlorinated solvent is dichloromethane.

39. The process of claim 36 or 37 wherein the aromatic hydrocarbon is toluene.

40. Amorphous rimonabant as obtained by the process of claims 36 to 39.

41. A pharmaceutical composition comprising one or more polymorphic Forms of rimonabant selected from the group consisting of Form III, Form IV, Form V or the amorphous as the active ingredient.

42. A method of treatment of obesity, smoking cessation, overweight and/or related diseases comprising administering to a patient in need of such treatment of a therapeutically effective amount of polymorphic form III, IV, V or the amorphous form of rimonabant.
